Europäisches Patentamt

European Patent Office

Office européen des brevets

(19)

(11) Numéro de publication : **0 032 872**
**B1**

(12) # FASCICULE DE BREVET EUROPÉEN

(45) Date de publication du fascicule du brevet :
**17.10.84**

(21) Numéro de dépôt : **81440002.4**

(22) Date de dépôt : **15.01.81**

(51) Int. Cl.³ : **A 61 J 3/00//** A61M5/00,
A61M16/00, A61M17/00

(54) **Procédé d'obtention d'un moyen thérapeutique, sous forme gazeuse, et moyen thérapeutique ainsi obtenu.**

(30) Priorité : **18.01.80 FR 8001366**

(43) Date de publication de la demande :
**29.07.81 Bulletin 81/30**

(45) Mention de la délivrance du brevet :
**17.10.84 Bulletin 84/42**

(84) Etats contractants désignés :
**AT DE GB NL**

(56) Documents cités :
  **FR-A- 19 168**
  **FR-A- 633 348**
  **FR-A- 733 447**
  **FR-A- 811 462**
  **FR-E- 30 903**
  **US-A- 3 192 106**

(73) Titulaire : **Fix, Roger**
**14, boulevard du Champ de Mars**
**F-68000 Colmar (FR)**

(72) Inventeur : **Fix, Roger**
**14, boulevard du Champ de Mars**
**F-68000 Colmar (FR)**

(74) Mandataire : **Nuss, Pierre**
**10, rue Jacques Kablé**
**F-67000 Strasbourg (FR)**

EP 0 032 872 B1

Jouve, 18, rue St-Denis, 75001 Paris, France

## Description

La présente invention concerne le domaine du traitement ou de la prévention de maladies chez l'homme, chez l'animal ou la plante, et a pour objet un procédé d'obtention d'un moyen thérapeutique, sous forme gazeuse.

L'invention a également pour objet le moyen thérapeutique ainsi obtenu.

Actuellement, les traitements thérapeutiques s'effectuent généralement au moyen de médicaments administrés sous forme liquide ou solide, par voie buccale ou rectale, ou encore par injection parentérale dans le cas de liquides.

Ces médicaments présentent généralement une bonne efficacité, mais, du fait de leur dosage, et de leur constitution, ils produisent le plus souvent des effets secondaires indésirables, voire même dangereux.

Il est également connu d'administrer, dans certains cas, des gaz purs ou en mélange, soit par les voies aériennes, notamment pour l'anesthésie, soit par injection intraveineuse, ou autre.

Ainsi, le US-A-3 192 106 décrit des mélanges gazeux contenant de l'oxyde azoteux et un procédé de réalisation de ces mélanges, qui consiste à introduire l'oxyde azoteux dans un récipient sous pression et à ajouter ensuite un gaz permanent jusqu'à obtention d'un mélange contenant 15 % à 75 % en volume d'oxyde azoteux. Ces gaz sont agités afin d'accélérer l'atteinte d'un équilibre. Ce procédé vise à réaliser un simple mélange intime de deux ou plusieurs gaz selon des proportions données, l'adjonction d'un gaz s'effectuant par intermittence et le brassage étant continu. Les mélanges gazeux ainsi obtenus sont utilisables pour l'anesthésie, ou à haute pression pour des essais d'étanchéité, mais ne peuvent être utilisés efficacement pour une action thérapeutique donnée.

Il est également connu, par le FR-A-19.168, un dispositif de mélange de gaz tels que de l'oxyde azoteux et des gaz oxygénés pour l'anesthésie. Un tel dispositif permet l'obtention en continu, en une opération unique, d'un mélange intime de gaz dans des proportions déterminées suivant les besoins. Cependant, les mélanges gazeux ainsi obtenus ne sont pas applicables efficacement dans des buts thérapeutiques.

Enfin, le FR-A-633.348, décrit un dispositif d'injection, dans des buts thérapeutiques, de petits volumes gazeux contenus dans des ampoules, ou analogues. Les gaz sont alors transférés des ampoules dans une seringue au moyen d'un robinet pouvant loger dans sa partie mobile, une ampoule dont les extrémités sont cassées lors de la manœuvre du robinet. Il est également prévu de transférer plusieurs gaz dans la seringue en vue de leur injection simultanée. Toutefois, un tel dispositif ne permet pas un mélange intime de plusieurs gaz, et l'efficacité thérapeutique du procédé est aléatoire.

La présente invention a pour but de pallier ces inconvénients.

Elle a, en effet, pour objet un procédé d'obtention d'un moyen thérapeutique, sous forme gazeuse, caractérisé en ce qu'il consiste essentiellement à diluer dans un récipient étanche, préalablement purgé, un gaz servant de remède dans un autre gaz, appelé gaz vecteur, suivant un rapport de dilution décimal, centésimal, ou autre, puis à dynamiser le mélange de gaz obtenu, pendant environ 5 minutes, par brassage, par chauffage du récipient, ou par circulation du mélange de gaz, à répéter les opérations de dilution et de dynamisation jusqu'à atteinte du degré de dilution désiré, qui correspond à une raréfaction du premier gaz dans le gaz vecteur, et enfin, à transvaser le moyen thérapeutique obtenu dans des moyens de transfert et d'application tels que des seringues étanches, des poires rectales étanches, ou des récipients étanches destinés à servir pour une inhalation ou à l'alimentation d'une seringue, ou une gélule pouvant être absorbée par voie orale.

Dans le cas d'un mélange des gaz en phase liquide, l'opération de transvasement du moyen thérapeutique dans les moyens de transfert et d'application, est précédée d'une phase de détente du mélange destinée à l'amener à l'état gazeux.

L'invention est mise en œuvre au moyen d'un dispositif, qui est essentiellement constitué par un récipient étanche muni d'un ou de plusieurs raccords pour des sources de gaz, par plusieurs sources de gaz, par un moyen de dynamisation tel qu'un ventilateur, un agitateur, une pompe de circulation reliée à deux extrémités du récipient, ou un dispositif de chauffage du récipient, par un moyen de transvasement du moyen thérapeutique dans les moyens de transfert et d'application, et, dans le cas d'une préparation en phase liquide, par un détendeur monté entre le récipient étanche et le moyen de transvasement.

L'invention a également pour objet, à titre de produit nouveau, un moyen thérapeutique, obtenu par application du procédé suivant la revendication 1 et caractérisé en ce qu'il est constitué par un gaz servant de remède, sous forme d'un gaz quelconque, même toxique, raréfié par dilution suivant un rapport décimal, centésimal, ou autre dans un autre gaz, appelé gaz vecteur, de préférence non toxique, la dilution obtenue étant dynamisée, en ce qu'il est renfermé dans un moyen de transfert et d'application tel qu'une seringue étanche, une poire rectale étanche, ou un récipient étanche destiné à servir pour l'inhalation ou l'alimentation d'une seringue, ou une gélule pouvant être absorbée par voie orale, et

(a) en ce que pour son application au traitement de l'asthme, il consiste en une dilution d'oxygène D6 dans de l'azote, ou

(b) en ce que pour son application au traitement de troubles de la concentration ou de la mémoire, il consiste en une dilution d'oxygène D6 ou de gaz carbonique D6 dans de l'azote, ou

(c) en ce que pour son application au traitement d'états de fatigue ou de problèmes de récupération, il consiste en une dilution d'ammoniac D6 dans de l'azote, de l'oxygène, ou de l'hydrogène.

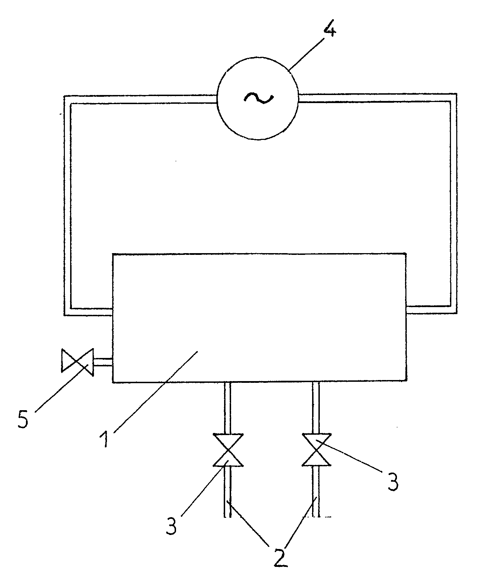

L'invention sera mieux comprise grâce à la description ci-après, qui se rapporte à un mode de réalisation préféré, donné à titre d'exemple non limitatif, et expliqué avec référence au dessin schématique annexé, dont la figure unique est une vue en coupe du dispositif pour la mise en œuvre du procédé conforme à l'invention.

Le procédé d'obtention d'un moyen thérapeutique consiste essentiellement à diluer dans un récipient étanche 1, préalablement purgé, un gaz servant de remède dans un autre gaz, appelé gaz vecteur, suivant un rapport décimal, centésimal, ou autre. Ces gaz sont introduits dans le récipient 1 à partir de sources de gaz 2 par l'intermédiaire de raccords 3. Après atteinte du premier degré de dilution, le mélange gazeux, qui est en phase liquide ou en phase gazeuse est dynamisé pendant environ 5 minutes au moyen d'une pompe de circulation 4 reliée aux extrémités du récipient 1. Puis une nouvelle dilution est réalisée suivie d'une nouvelle dynamisation, et ces opérations sont répétées jusqu'à atteinte du degré de dilution désiré. Le moyen thérapeutique ainsi obtenu est alors transvasé, s'il est en phase gazeuse, par l'intermédiaire d'un moyen de transvasement 5 tel qu'un robinet, ou analogue, dans un moyen de transfert et d'application tel qu'une seringue étanche, une poire rectale étanche, ou un récipient étanche destiné à servir pour une inhalation ou à l'alimentation d'une seringue, ou une gélule pouvant être absorbée par voie orale.

Dans le cas d'une préparation de la dilution en phase liquide, il est prévu, entre le récipient 1 et le moyen de transvasement 5, un détendeur, non représenté, amenant le mélange en phase gazeuse.

Le gaz servant de remède, qui peut être constitué par n'importe quel gaz, même par un gaz toxique, est dilué dans un autre gaz, de préférence non toxique, suivant les proportions 1/9, 1/99, ou autres. Ainsi, à titre d'exemple, 1 litre d'oxygène mélangé avec 9 litres de gaz carbonique fournira de l'oxygène dilué au dixième, de sorte que l'on obtient la première dilution décimale de l'oxygène, appelée par abréviation de l'oxygène D1. Si 1 litre de ce premier mélange d'oxygène D1 est à nouveau dilué dans 9 litres de gaz carbonique, on obtient de l'oxygène D2. Par répétition de ces dilutions on obtiendra de l'oxygène D3, D4, etc.

Dans le cas d'un rapport de dilution de 1/99, on obtient des dilutions centésimales de l'oxygène, qui sont notées successivement oxygène C1, C2, etc.

Les gaz utilisés peuvent aussi bien être des gaz purs que des mélanges complexes de gaz obtenus par divers procédés, tels que putréfaction, distillation, fermentation, combustion, carbonisation, évaporation, sublimation, ou autre.

L'opération de dynamisation exécutée après chaque dilution est destinée à soumettre le mélange de gaz à un brassage vigoureux de manière à le rendre parfaitement homogène.

Un tel procédé de raréfaction d'un gaz dans un autre par étapes successives séparées chaque fois par une opération de dynamisation est absolument indispensable pour obtenir l'efficacité spécifique du moyen thérapeutique, la succession de ces opérations conférant une activité particulière audit moyen thérapeutique.

Le moyen thérapeutique conforme à l'invention peut être appliqué dans un vaste domaine de la pathologie, et est avantageusement conditionné dans des seringues étanches, dans des poires rectales étanches, ou dans des récipients sous forme de flacons étanches pouvant être ouverts pour un traitement par inhalation, ou présentant un opercule souple pouvant être percé au moyen de l'aiguille d'une seringue en vue du remplissage de cette dernière, ou encore dans des bouteilles sous pression, ou dans des gélules destinées à une absorption par voie orale.

A titre d'exemple d'application thérapeutique, il est possible de soigner l'asthme avec une dilution d'oxygène D6 dans de l'azote.

Le remède ainsi obtenu par raréfaction de l'oxygène dans de l'azote pris comme vecteur jusqu'à obtention d'un mélange gazeux definitif comportant un volume d'oxygène pour un million de volumes d'azote présente une très grande efficacité contrairement à un mélange simple en une opération unique des deux composants, qui aboutit à une substance n'ayant pas l'action thérapeutique désirée.

Les troubles de la concentration ou de la mémoire peuvent être soignés avec de l'oxygène D6 ou du gaz carbonique D6 dans de l'azote.

La fatigue et les problèmes de récupération peuvent être traités à l'ammoniac D6 dans un diluant azote, oxygène, hydrogène, ou autre.

A titre d'exemple, l'oxygène préparé selon le procédé conforme à l'invention présente la propriété pharmacologique essentielle d'agir sur le métabolisme de l'oxygène dans l'organisme c'est-à-dire sur la respiration cellulaire.

Le moyen thérapeutique conforme à l'invention, présente l'avantage, par rapport aux remèdes dilués et dynamisés existant, préparés à partir de substances solides ou liquides, d'une plus grande efficacité et de ne pas occasionner d'effets secondaires indésirables.

Bien entendu, l'invention n'est pas limitée au mode de réalisation décrit et représenté au dessin annexé.

**Revendications**

1. Procédé d'obtention d'un moyen thérapeutique, sous forme gazeuse, caractérisé en ce qu'il consiste essentiellement à diluer dans un récipient étanche (1), préalablement purgé, un gaz servant de remède dans un autre gaz, appelé gaz vecteur, suivant un rapport de dilution décimal, centésimal, ou autre, puis à dynamiser le mélange de gaz obtenu, pendant environ 5 minutes, par brassage, par chauffage du récipient, ou par circulation du mélange de gaz, à répéter les opérations de dilution et de dynamisation jusqu'à atteinte du degré de dilution désiré, qui correspond à une raréfaction du premier gaz dans le gaz vecteur, et enfin, à transvaser le moyen thérapeutique obtenu dans des moyens de transfert et d'application tels que des seringues étanches, des poires rectales étanches, ou des récipients étanches destinés à servir pour une inhalation ou à l'alimentation d'une seringue, ou une gélule pouvant être absorbée par voie orale.

2. Moyen thérapeutique, obtenu par application du procédé suivant la revendication 1, caractérisé en ce qu'il est constitué par un gaz servant de remède, sous forme d'un gaz quelconque, même toxique, raréfié par dilution suivant un rapport décimal, centésimal, ou autre, dans un autre gaz, appelé gaz vecteur, de préférence non toxique, la dilution obtenue étant dynamisée, en ce qu'il est renfermé dans un moyen de transfert et d'application tel qu'une seringue étanche, une poire rectale étanche, ou un récipient étanche destiné à servir pour l'inhalation ou l'alimentation d'une seringue, ou une gélule pouvant être absorbée par voie orale, et (a) en ce que pour son application au traitement de l'asthme, il consiste en une dilution d'oxygène D6 dans de l'azote, ou (b) en ce que pour son application au traitement de troubles de la concentration ou de la mémoire, il consiste en une dilution d'oxygène D6 ou de gaz carbonique D6 dans de l'azote, ou (c) en ce que pour son application au traitement d'états de fatigue ou de problèmes de récupération, il consiste en une dilution d'ammoniac D6 dans de l'azote, de l'oxygène, ou de l'hydrogène.

## Claims

1. Process for obtaining a therapeutic agent in gaseous form, characterized in that it essentially comprises diluting in a previously purged, airtight container (1) a gas serving as a remedy in another gas, called the vector gas, in accordance with a decimal, centesimal or other dilution ratio, followed by concentrating the gaseous mixture obtained for approximately 5 minutes by stirring, by heating the container or by circulation of the gaseous mixture and repeating the dilution and concentration operations until the desired degree of dilution is reached, which corresponds to a rarefaction of the first gas in the vector gas and finally transferring the therapeutic agent obtained into transfer and application means such as airtight syringes, airtight rectal bulbs or airtight containers intended to serve for an inhalation or for the feeding of a syringe, or an orally absorbable gelatin capsule.

2. Therapeutic agent obtained by applying the process according to claim 1, characterized in that it is constituted by a gas serving as a remedy, in the form of a random and even toxic gas, rarefied by dilution in a decimal, centesimal or other ratio in another gas, called the vector gas, which is preferably non-toxic, the dilution obtained being concentrated, in that it is contained in a transfer and application means such as an airtight syringe, an airtight rectal bulb or an airtight container used for inhalation or for feeding a syringe, or an orally absorbable gelatin capsule, and in that (a) when used in the treatment of asthma it consists of a D6 oxygen dilution in nitrogen, or (b) when used in the treatment of memory or concentration disorders it consists of a D6 oxygen or D6 carbon dioxide gas dilution in nitrogen, or (c) when used in the treatment of fatigue states or recovery problems it consists of a D6 ammonia dilution in nitrogen, oxygen or hydrogen.

## Ansprüche

1. Verfahren zur Herstellung eines therapeutischen Mittels in einem gasförmigen Zustand, dadurch gekennzeichnet, dass man im wesentlichen in einem vorher ausgeblasenen luftdichten Gefäss (1) ein heilwirksames Gas, mit einem weiteren, hier Trägergas genannten Gas mit einem dezimalen, zentesimalen, oder sonstigen Verhältnis verdünnt, dass man dieses Gasgemisch ca. 5 Minuten lang durch Wirbelung, durch Erhitzen des Gefässes oder durch Umwälzung dynamisiert, dass das Verdünnen und das Dynamisieren solange wiederholt werden, bis der gewünschte Verdünnungsgrad erreicht wird, der einer Verdünnung des ersten Gases im Trägergas entspricht, und dass schliesslich das therapeutische Mittel in eine Übertragungs- und Applikationsform gebracht wird, die z. B. in Gestalt einer luftdichten Injektionsspritze, eines luftdichten Rektalovulums, oder eines abgedichteten Behälters zur Verabreichung des Mittels durch Einatmung oder zur Aufnahme in eine Injektionsspritze, oder als eine oral resorbierbare Kapsel vorliegt.

2. Nach dem Verfahren gemäss Anspruch 1 erhaltenes therapeutisches Mittel, dadurch gekennzeichnet, dass es aus einem heilwirksamen Gas besteht, das in der Form eines beliebigen, auch toxischen, mit einem dezimalen, zentesimalen oder sonstigen Verhältnis mit einem weiteren als Trägergas dienenden und vorzugsweise nicht toxischen Gas verdünnt vorliegt, wobei das Verdünnungsgemisch dynamisiert ist, dass das Mittel in eine Übertragungs- und Applikationsform gebracht wird, die z. B. in der Form einer luftdichten Injektionsspritze, eines luftdichten Rektalovulums, oder eines abgedichteten Behälters zur Verabreichung des Mittels durch Einatmung oder zur Aufnahme in eine Injektionsspritze, oder als eine oral zu verabreichende Kapsel vorliegt, und dass (a) zur Verabreichung bei der Asthmabehandlung es aus einem Gemisch besteht, in dem Sauerstoff mit einer D6-Verdünnung in Stickstoff vorliegt, oder (b) zur Verabreichung bei der Behandlung von Konzentrations- oder Gedächtnisschwäche es aus einem Gemisch besteht, in dem Sauerstoff oder Kohledioxid mit D6-Verdünnung in Stickstoff vorliegt, oder (c) zur Verabreichung bei der Behandlung von Erschöpfungszuständen oder im Falle von Rekonvaleszenzproblemen es aus einem Gemisch besteht, in dem Ammoniak mit D6-Verdünnung in Stickstoff, Sauerstoff oder Wasserstoff vorliegt.